# EUROPEAN PATENT SPECIFICATION

(11) **EP 3 834 782 B1**
(45) Date of publication and mention of the grant of the patent: **03.07.2024**
(21) Application number: 19847979.2
(22) Date of filing: 09.08.2019
(51) Int. Cl.: A61F 2/966, A61F 2/954, A61F 2/06

(54) **DELIVERY APPARATUS**
FREISETZUNGSVORRICHTUNG
APPAREIL DE POSE

(30) Priority: 09.08.2018 CN 201810899886; 09.08.2018 CN 201821276778 U; 09.08.2018 CN 201810899863; 09.08.2018 CN 201821276305 U
(43) Date of publication of application: 16.06.2021
(73) Proprietor: Hangzhou Endonom Medtech Co., Ltd, Hangzhou, Zhejiang 310051 (CN)
(72) Inventor: WANG, Yongsheng, Hangzhou, Zhejiang 310051 (CN); WU, Shichao, Hangzhou, Zhejiang 310051 (CN); LI, Jianmin, Hangzhou, Zhejiang 310051 (CN)
(74) Representative: HWP Intellectual Property
(86) International application number: PCT/CN2019/099899
(87) International publication number: WO 2020/030075

(56) References cited:
- EP-A1- 2 958 528
- EP-A1- 3 315 101
- EP-B1- 2 139 429
- CN-A- 103 860 292
- CN-A- 104 254 301
- CN-A- 107 280 808
- CN-U- 206 424 184
- US-A1- 2011 125 244
- US-A1- 2011 270 375
- US-A1- 2015 305 902
- US-A1- 2018 036 158

## Description

### TECHNICAL FIELD

The present invention relates to a field of medical devices, and particularly to a delivery device.

### BACKGROUND

Abdominal aortic aneurysm (AAA) is a typical aortic disease. Epidemiological studies show that the incidence of AAA in men is about 5%, while with respect to patients in and over the age of 80, the incidence is increased to about 10%, and they have a higher risk of tumor rupture than the other people. It is predicted that by 2020, there will be 30 million people over the age of 80 and more than 12 million people over the age of 90 in China, which means that more and more elderly patients suffering AAA will need treatment in the future.

Exiting methods for the treatment of abdominal aortic aneurysms mainly includes traditional open surgery and abdominal aortic endovascular repair (EVAR), both of which have their own advantages. Since the first case of abdominal aortic endovascular repair has been used in the treatment of abdominal aortic aneurysms in the 1990s, because of its advantages of low trauma, short operation and hospital stay, fast postoperative recovery, low perioperative mortality and complication rate, etc., it is rapidly developing in just 20 years.

The abdominal aortic endovascular repair has its major advantages in no need for thoracotomy and laparotomy, no need for clamping and blocking blood vessels, no ischemia of internal organs, and fewer complications. However, the biggest restriction on the abdominal aortic endovascular repair is that it is impossible to cover the splanchnic artery, especially the superior mesenteric artery and the renal artery. The difficulty still lies in the abdominal aortic endovascular repair involving the renal artery.

At present, the "fenestrated stent-graft" is adopted to carry out the stent-graft implantation treatment for such patients. In other words, a main stent provided with a fenestration is implanted in the abdominal aorta, and a branch stent is implanted through the fenestration to lead to the branch vessel. The stent-graft implanted for endovascular treatment of abdominal aortic aneurysms involves the reconstruction of four branch arteries including the celiac artery, superior mesenteric artery and the left and right renal arteries.

In the existing technology, after the main stent is implanted into the artery blood vessel, a repeated operation is required to implant the branch stent. Before the branch stent is implanted, a branch guidewire needs to be guided into the branch vessel through the fenestration of the main stent, and the branch stent is then guided by the branch guidewire into the branch vessel through the fenestration of the main stent. However, it is very difficult to guide the branch guidewire into the branch vessel, as the branch guidewire needs to pass through the fenestration of the main stent to reach the branch vessel, and it is difficult for a distal end of the branch guidewire to be aligned with and pass through the fenestration of the main stent. How to effectively reduce the difficulty for the branch guidewire reaching the branch blood vessel through the fenestration is a problem needed to be solved jointly by both medical personnel and medical researchers.

For example, US 2011/0270375 A1 describes an introducer for an iliac side branch device, the introducer comprises an introducer catheter extending over a guide wire catheter. The guide wire catheter extends from the distal end of the introducer to immediately distal of a nose cone dilator. A branched stent graft is retained at its proximal end onto the introducer immediately distal of the nose cone dilator. The branched stent graft is retained at its distal end onto the introducer. A sleeve operated by a sleeve manipulator is mounted on the introducer catheter, the sleeve extends over the branched stent graft to the nose cone dilator. A handle is arranged at the distal end of the introducer catheter. An indwelling catheter enters a lumen of the introducer catheter at the handle and exits from the introducer catheter at the distal end of the branched stent graft. Wherein within the indwelling catheter, there is a guide wire.

US 2018/0036158 A1 describes a medical-device position adjusting method and medical device system, wherein the medical device system comprises a medical device; a guide wire that is used for the medical device; an anchor device that fixes a distal portion of the guide wire to the inside of the blood vessel, an anchor-device guide wire that is used for the anchor device, a guiding catheter that introduces the medical device and the anchor device inside the blood vessel, and a proximal-end fixing member that fixes the guiding catheter and the guide wire when the position of the guide wire is changed, such that the guide wire can move in an insertion/pulling-out direction.

EP 2 958 528 B1 describes a stent-graft delivery system having a tip capture mechanism with elongated cables for gradual deployment and repositioning, wherein the stent-graft delivery system comprises: A elongate inner shaft having a handle coupled to a proximal end thereof and a distal tip assembly coupled to a distal end thereof. A self-expanding stent-graft prosthesis is provided inside the distal tip assembly, and a plurality of elongate cables extend along the inner shaft to engage and lock the self-expanding stent-graft. A guiding assembly is slidingly disposed over inner shaft for guiding and directing cables.

US 2015/0305902 A1 describes a delivery system for a retractable outer sheath, wherein the delivery system comprises a handle assembly, an inner shaft, and an outer retractable sheath or cover. The handle is disposed over the slide shaft and is movable relative to the slide shaft. The outer sheath is coupled to the handle, and the guide wire runs through the outer sheath. The position of the outer sheath can be adjusted by advancing or retracting the handle relative to the slide shaft.

Finally, US 2011/0125244 A1 describes a stent graft and introducer assembly, wherein the introducer includes an external manipulation section, a distal attachment region and a proximal attachment region. The stent graft is retained in a compressed condition by a sheath, and the sheath is connected to the external manipulation section via a seal section and a pusher rod. A plurality of trigger wires is provided along the pusher rod and the sheath to connect to the diameter-reducing suture loops of the stent graft. Therefore, by adjusting the trigger wires, the stent graft can be released in sequence and be adjusted at the same time.

### SUMMARY

The present invention aims to solve the aforementioned technical problems by providing a delivery device that decreases difficulty for a branch guidewire passing through a fenestration of a main stent to reach a branch vessel. To this end, the above problem is solved by a delivery device according to independent claim 1. Further preferred embodiments result from the following description, the dependent claims and the drawings.

In order to solve the aforementioned technical problems, the present invention provides a delivery device configured to deliver a main stent having a fenestration, wherein the delivery device includes:
a core assembly;
an outer sheath, which is hollow and mounted around an outer periphery of the core assembly, with a delivering gap defined between the outer sheath and the core assembly, wherein the delivering gap has a distal end for receiving a folded main stent; and
a pre-embedded guidewire, entering the delivering gap through the proximal end of the delivering gap and extending to the distal end of delivering gap passage, the pre-embedded guidewire having a distal end configured to enter into the main stent from outside of the main stent via the fenestration, and the pre-embedded guidewire being configured to guide a branch guidewire to pass through the main stent from an inside to the outside of the main stent via the fenestration of the main stent.

According to the invention, the delivery device further includes a control handle connected with the outer sheath, and wherein the control handle controls the outer sheath to move axially relative to the core assembly so as to enable the folded main stent to be in a partly released state or in a completely released state.

Further according to the invention, a sheath joint is secured to an outer periphery of the outer sheath, and the control handle includes:
a support body, with the sheath joint provided therein, wherein when being subjected to an axial force, the sheath joint moves axially within the support body to drive the outer sheath to move axially;
a fixed handle, mounted outside a distal end of the support body and secured thereto; and
a sliding handle, mounted outside a distal end of the support body adjacent to a proximal end of the fixed handle, the sliding handle being rotatable at the outside of the support body so as to drive the sheath joint to move axially.

Finally according to the invention, the sliding handle is axially slidable on the support body, an unlocking button is embedded in the fixed handle, the unlocking button extends to the side of the sliding handle with a hook, and the hook hooks the sliding handle in such a way that the sliding handle is arranged next to the fixed handle and prevented from axially sliding.

In one preferred embodiment of the present disclosure, a locking assembly is provided at a proximal end of the delivery device and configured to lock a movement of the pre-embedded guidewire.

In one preferred embodiment of the present disclosure, the delivery device further includes:
the control handle, connected with a proximal end of the outer sheath;
a rear fastener, secured to a proximal end of the control handle; and
a rear slideway, secured to a proximal end of the rear fastener, wherein the locking assembly is provided on the rear slideway, and the pre-embedded guidewire passes through the rear slideway and the rear fastener and enters the delivering gap.

In one preferred embodiment of the present disclosure, the rear slideway defines therein a hollow rear sliding passage extending in an axial direction of the rear slideway, the rear sliding passage communicates with the delivering gap; the locking assembly comprises a wire fastener, a fixed annular protrusion secured on the rear slideway and a blocking member secured inside the rear sliding passage, the pre-embedded guidewire enters the rear sliding passage from a gap between the wire fastener and the blocking member, the wire fastener and the fixed annular protrusion are connected by a threaded connection, and wherein when the wire fastener presses tightly the pre-embedded guidewire onto the blocking member, the movement of the pre-embedded guidewire is locked.

In one preferred embodiment of the present disclosure, the delivery device further includes a stent restraining assembly configured to restrain a released part of the main stent to be partly unfolded when the main stent is in the partly released state in such a way to restrain an outer diameter of the released part of the main stent, and to make the main stent be fully unfolded when the main stent is in the completely released state.

In one preferred embodiment of the present disclosure, the stent restraining assembly includes at least one control wire, and the control wire enters the delivering gap through a proximal end of the delivering gap and extending to the distal end of the delivering gap, and wherein the control wire has a distal end configured to circumferentially restrain the released part of the main stent.

In one preferred embodiment of the present disclosure, the stent restraining assembly further includes a pull-tab secured to a proximal end of the control wire, the pull-tab is configured to apply a force to a proximal end of the delivery device so as to release the restraining of the control wire to the main stent.

In one preferred embodiment of the present disclosure, the delivery device further includes another locking assembly configured to lock the movement of the stent restraining assembly to avoid releasing the restraining of the control wire to the main stent unintentionally.

In one preferred embodiment of the present disclosure, an elongated hole is defined on the support body and extends in an axial direction, a tooth block is mounted outside the support body at a position corresponding to the elongated hole, the sheath joint comprises a joint body, and an abutment block and a distal protrusion provided on the joint body, the abutment block and the distal protrusion extend through the elongated hole and abut against a proximal end and a distal end of the tooth block, respectively, in such a way to limit an axial movement of the tooth block relative to the sheath joint, the sliding handle is provided with an internal thread, the tooth block is provided with an external thread engaging with the internal thread, and wherein when the sliding handle rotates, the sliding handle drives the sheath joint to move axially through the tooth block, the abutment block and the distal protrusion, and in turn drives the outer sheath to move axially.

In one preferred embodiment of the present disclosure, the delivery device further includes a push-rod, wherein the push-rod has a distal end disposed in the delivering gap, and the distal end of the push-rod is configured to abut against the main stent when the control handle controls the outer sheath to axially move relative to the core assembly towards the proximal end, so as to prevent the main stent from moving towards the proximal end of the delivering gap.

In one preferred embodiment of the present disclosure, the delivery device further includes a support tube, wherein the support tube has a distal end disposed in the delivering gap, and the push-rod is disposed in the support tube.

In one preferred embodiment of the present disclosure, the push-rod defines therein a through passage extending in an axial direction thereof, and the pre-embedded guidewire enters the through passage from a proximal end thereof and extends out of the through passage from a distal end thereof.

The embodiments of the present disclosure have advantages as follows.

The delivery device includes a pre-embedded guidewire that enters the proximal end of the delivering gap and extends to the distal end of the delivering gap. The pre-embedded guidewire has a distal end configured to enter into an inside of the main stent from an outside thereof via a fenestration. The pre-embedded guidewire is configured to guide a branch guidewire to pass through the main stent from an inside to the outside thereof via the fenestration. In this way, with the guide of the pre-embedded guidewire, the branch guidewire may easily pass through the fenestration of the main stent. Thereafter, the branch guidewire may be further delivered to easily enter into the branch vessel. Thereafter, the branch stent may easily pass through the fenestration of the main stent and then reach the branch vessel with the guide of the branch guidewire, thus, facilitating the delivery of the branch stent.

### BRIEF DESCRIPTION OF THE DRAWINGS

In order to more clearly illustrate the technical solutions of the embodiments according to the present application or the existing technology, drawings used in the description of the embodiments according to the present application or the existing technology will be briefly introduced below. It should be appreciated that the drawings described below merely illustrate some embodiments of the present application, and other drawings may be obtained by those skilled in the art without departing from the scope of the drawings.
FIG. 1 is a perspective view of a delivery device according to an embodiment of the present disclosure.
FIG. 2 is a cross-sectional view of the delivery device according to the embodiment of the present disclosure, viewed from one direction.
FIG. 3 is a cross-sectional view of the delivery device according to the embodiment of the present disclosure viewed from another direction.
FIG. 4 is an enlarged view of a circled portion A of Figure 2.
FIG. 5 is an enlarged view of a circled portion B of Figure 2.
FIG. 6 is an enlarged view of a circled portion D of Figure 3.
FIG. 7 is an enlarged view of a circled portion C of Figure 2.
FIG. 8 is an enlarged view of a circled portion E of Figure 3.
FIG. 9 is a schematic view of the delivery device according to the embodiment of the present disclosure, viewed from one aspect, in which a main stent is in a partly released state.
FIG. 10 is a schematic view of the delivery device according to the embodiment of the present disclosure, viewed from another aspect, in which the main stent is in a partly released state.
FIG. 11 is a schematic view showing the main stent according to the embodiment of the present disclosure in a completely released state.
FIG. 12 is a schematic view showing the main stent according to the embodiment of the present disclosure which is completely released in an arterial blood vessel.
FIG. 13 is a schematic view showing the embodiment of the present disclosure, in which a branch guidewire passes through the main stent disposed in the arterial blood vessel and reaches a branch vessel.
FIG. 14 is a schematic diagram showing the embodiment of the present disclosure, in which a branch stent passes through the main stent disposed in the arterial blood vessel and reaches a branch vessel over the branch guidewire.

### Reference numerals shown in the drawings.

1000 - delivery device; 100 - core assembly; 110 - inner core; 120 - outer tube; 130 - leading head; 131 - internal lumen; 140 - stent securing assembly; 142 - positioning sleeve; 143 - securing anchor; 150 - outer tube fastener; 160 - inner core securing sleeve; 170 - rear releasing knob; 200 - outer sheath; 210 - delivering gap; 220 - sheath joint; 221 -abutment block; 222 - joint body; 223 - distal protrusion; 300 - control handle; 310 - support body; 311 - elongated hole; 320 - fixed handle; 321 - unlocking button; 322 - hook; 323 - button support; 324 - positioning post; 330 - sliding handle; 332 - rotating knob; 333 - inner flange; 334 - friction-reducing protruding ring; 340 -tooth block; 400 - stent restraining assembly; 410 - control wire; 420 - pull-tab; 500 - first locking assembly; 510 - pull-tab fastener; 520 - proximal releasing knob; 610 - push-rod; 611 - through passage; 615 - rear sliding passage; 620 - support tube; 630 - push-rod fastener; 640 - rear fastener; 650 - rear slideway; 670 - luer joint; 680 - outer cover; 710 - pre-embedded guidewire; 720 - second locking assembly; 721 - wire fastener; 722 - fixed annular protrusion; 723 - blocking member; 800 - main stent; 810 - tubular membrane; 811 -fenestration; 820 - annular support frame; 830 - connecting member; 840-bare stent; 900 - branch stent; 910 - branch guidewire; 2000 - arterial blood vessel; 2001 - one end of the arterial blood vessel; 2002 - the other end of the arterial blood vessel; 2100 - branch vessel; 3001 - TPU flexible tube; 3002 - three-way valve.

### DESCRIPTION OF THE EMBODIMENTS

The technical solutions in the embodiments of the present disclosure will be clearly and completely described below in conjunction with the accompanying drawings in the embodiments of the present disclosure. Obviously, the described embodiments are only a part of the embodiments of the present disclosure, not all of the embodiments. Based on the embodiments of the present disclosure, all other embodiments obtained by those skilled in the art without inventive work shall fall within the protection scope of the present disclosure.

The terms "include", "comprise", "have" and any variations thereof in the specification, claims and drawings of the present disclosure are intended to cover non-exclusive inclusions. For example, a process, method, system, product or device including a series of steps or elements, is not limited to the steps or elements listed herein, but optionally includes other steps or elements that are not listed herein, or optionally further includes steps or elements inherent to such process, method, product or device. Furthermore, the terms "first," "second," and "third," etc. are used to distinguish between different objects and are not used to describe a particular order.

For clarity of description, one end of the delivery device close to the operator is defined as a proximal end of the delivery device, and the other end relatively far from the operator is defined as a distal end of the delivery device; one end of the main stent close to the patient's heart is defined as a proximal end of the main stent, and the other end relatively far from the heart is defined as a distal end of the main stent. The terms "proximal end" and "distal end" for the delivery device and the main stent are defined according to different references.

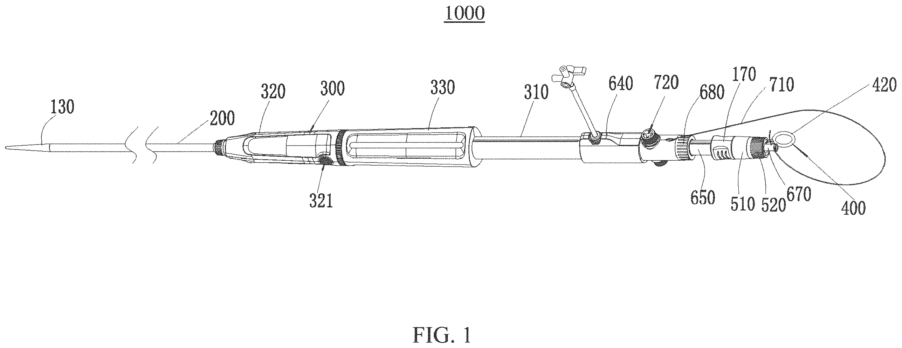

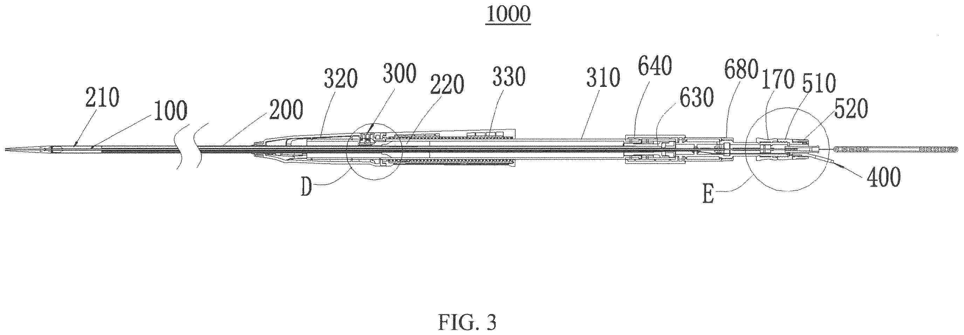

A delivery device is provided according to an embodiment of the present disclosure, which is configured to deliver a main stent. Herein, the main stent is loaded within the delivery device and provided with one or more fenestrations. Referring to FIGs. 1 to 3, the delivery device 1000 includes a core assembly 100, an outer sheath 200 and a pre-embedded guidewire 710.

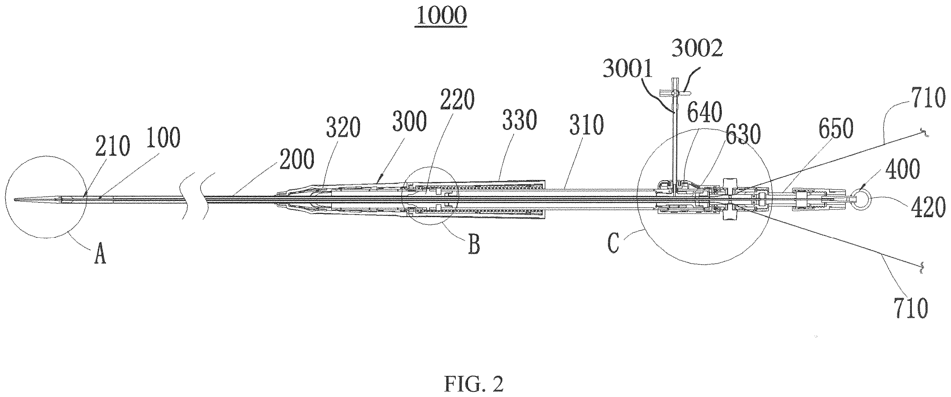

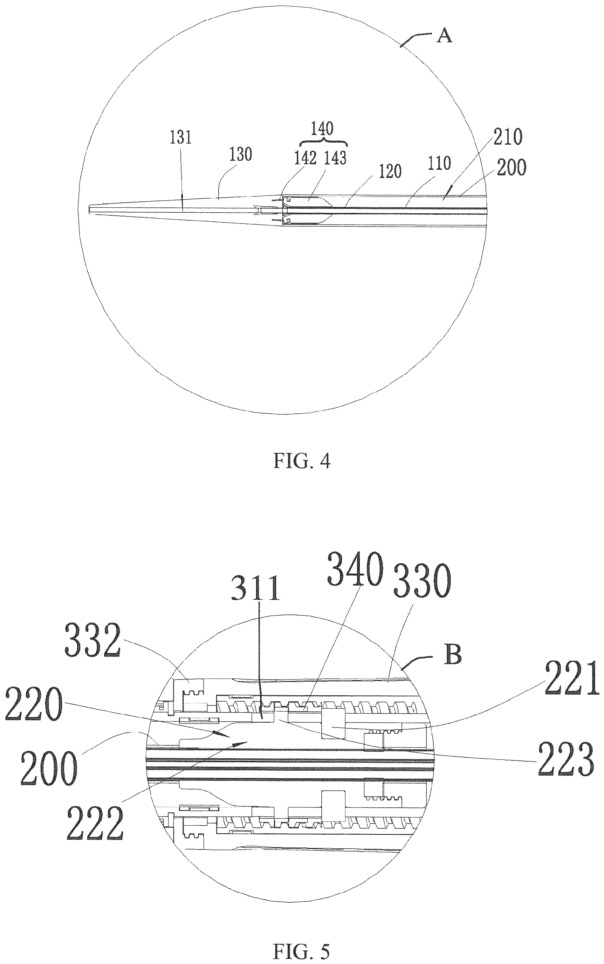

Specifically, in this embodiment, referring to FIGs. 2 and 4, the core assembly 100 includes an inner core 110 and an outer tube 120. The outer tube 120 is hollow and mounted around the inner core 110. The outer tube 120 is slidable relative to the inner core 110 in an axial direction.

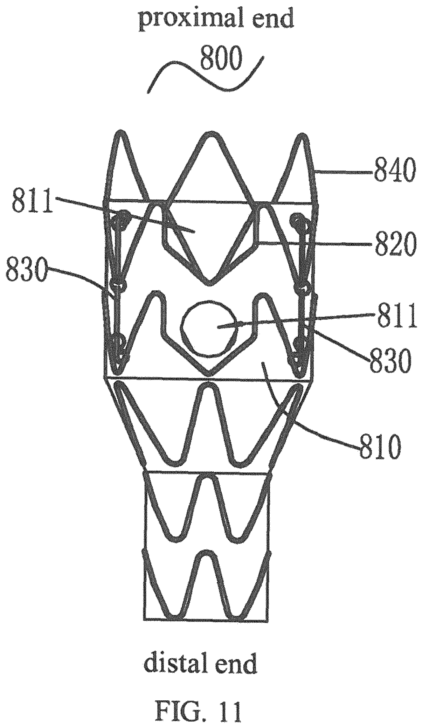

In this embodiment, the delivery device 1000 further includes a leading head 130 and a stent securing assembly 140. The leading head 130 is conical and has a distal end being cuspidal. The leading head defines an internal lumen 131 in a center thereof in the axial direction. The inner core 110 has a distal end secured to a proximal end of the leading head 130. The inner core 110 is hollow and communicates with the internal lumen 131 of the leading head 130. The stent securing assembly 140 is provided adjacent to the proximal end of the leading head 130. The stent securing assembly 140 includes a positioning sleeve 142 and a securing anchor 143. The securing anchor 143 is secured to a distal end of the outer tube 120. The securing anchor 143 is columnar and provided with a plurality of protrusions evenly spaced from one another in a circumference thereof for securing a bare stent 840 (as shown in FIG. 11) at a proximal end of the main stent 800, so as to position the proximal end of the main stent 800. That is, the proximal end of the main stent 800 surrounds the securing anchor 143 and is connected thereto. The positioning sleeve 142 has a distal end connected to the leading head 130 by molding. The positioning sleeve 142 is hollow and has a proximal end mounted around the securing anchor 143. A limiting gap is defined between the securing anchor 143 and an inner wall of the positioning sleeve 142, and the proximal end of the main stent 800 is disposed in the limiting gap.

In this embodiment, the outer sheath 200 is hollow and is mounted around an outer periphery of the core assembly 100. Specifically, the outer sheath 200 is mounted around an outer periphery of the outer tube 120, and is slidable relative to the outer tube 120 in an axial direction. A delivering gap 210 is defined between the outer sheath 200 and the core assembly 100. Specifically, the delivering gap 210 is defined between the outer sheath 200 and the outer tube 120. Herein, the delivering gap 210 refers to a space between an inner wall of the outer sheath 200 and an outer wall of the outer tube 120. The whole securing anchor 143 and the proximal end of the positioning sleeve 142 are disposed in the delivering gap 210. The delivering gap 210 has a distal end for receiving the folded main stent 800. Herein, the main stent 800 is forced by an external force to have a compressed profile so as to be received in the delivering gap 210. The bare stent 840 at the proximal end of the main stent 800 is positioned on the protrusions of the securing anchor 143. The main stent 800 is completely received in the delivering gap 210.

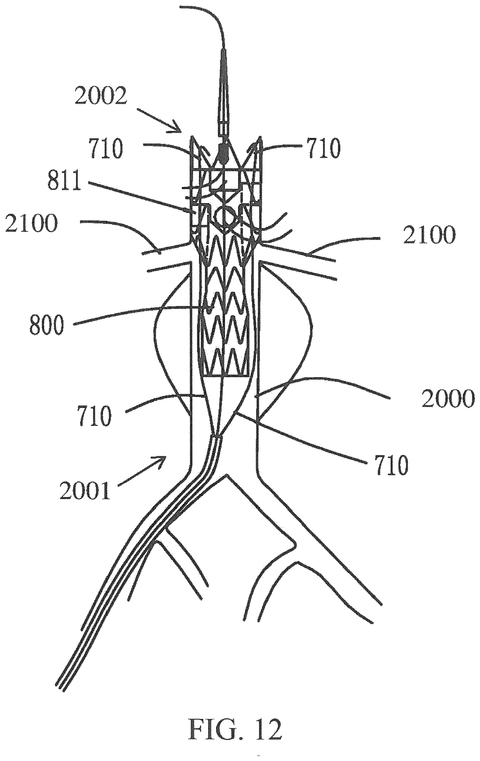

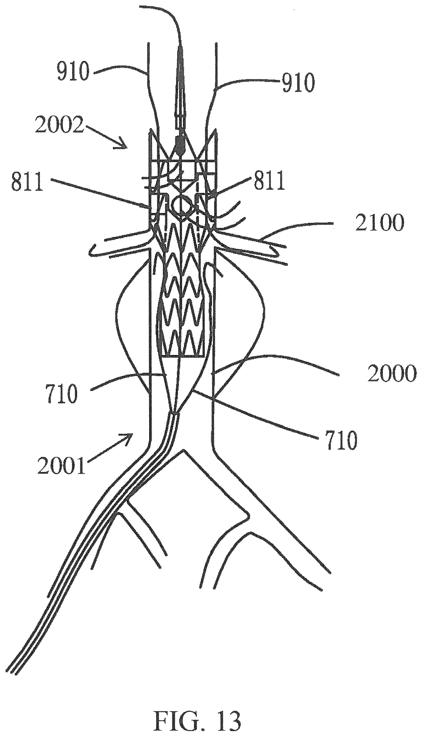

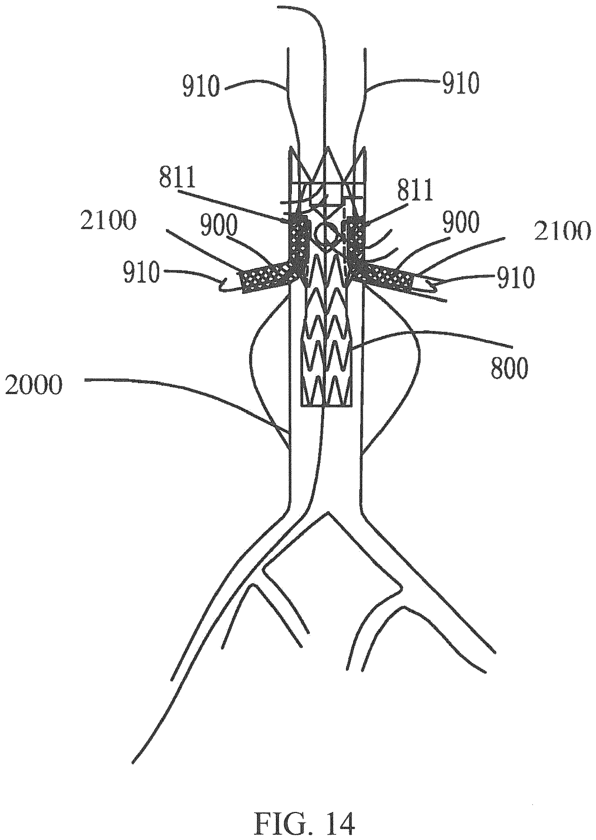

In this embodiment, as shown in FIGs. 1, 2 and 4, during use, the pre-embedded guidewire 710 enters the proximal end of the delivering gap 210 and extends to the distal end of the delivering gap 210. As shown in FIGs. 11 to 12, before the main stent 800 is positioned on the protrusions of the securing anchor 143, the pre-embedded guidewire 710 is advanced forwards and the distal end of the pre-embedded guidewire 710 enters into the main stent 800 from an outside thereof via the fenestration 811 and then is positioned inside the main stent 800. Thereafter, the surgeon compresses the main stent 800 and places it into the delivering gap 210, and then pull backwards the pre-embedded guidewire which is partly loosed with the distal end of the pre-embedded guidewire 710 remaining stationary, that is, the distal end of the pre-embedded guidewire 710 remains being positioned inside the main stent 800. Thereafter, the surgeon delivers the delivery device 1000 into the arterial blood vessel 2000 of the patient from one end 2001 of the arterial blood vessel 2000. When the main stent 800 in the delivery device 1000 reaches a pre-determined position, the main stent 800 is partly released from the delivery device 1000, such that the main stent 800 is in the partly released state. Alternatively, in other embodiments, the main stent 800 may be completely released. At that time, the fenestration 811 on the main stent 800 is exposed. The surgeon may adjust the position of the fenestration 811, etc. by moving the delivery device 100 axially or circumferentially, for example, to adjust the fenestration 811 to make it be aligned with the branch vessel 2100. Of course, the surgeon may not carry out the adjustment. Thereafter, the pre-embedded guidewire 710 is delivered towards the distal end of the delivery device 1000 by the surgeon and advances forwards until it extends out from the other end 2002 of the arterial blood vessel 2000. Thereafter, as shown in FIG. 13, a branch guidewire 910 is connected to the pre-embedded guidewire 710 by the surgeon at the other end 2002 of the arterial blood vessel 2000, and the branch guidewire 910 is drawn by the surgeon through the pre-embedded guidewire 710. For example, the distal end of the pre-embedded guidewire 710 may be provided with a bending structure, by which the branch guidewire 910 is hooked, to thereby achieve drawing the branch guidewire via the pre-embedded guidewire 710. Specifically, the pre-embedded guidewire 710 is pulled by the surgeon towards the proximal end of the delivery device 1000. With the guide of the pre-embedded guidewire 710, the branch guidewire 910 passes through the main stent 800 from the inside thereof to the outside thereof via the fenestration 811 of the main stent 800, and then enters the branch vessel 2100. After the branch guidewire 910 enters the branch vessel 2100, the pre-embedded guidewire is withdrawn towards the end 2001 of the arterial blood vessel 2000, such that the pre-embedded guidewire 710 is detached from the branch guidewire 910. For example, the hook connection between the bending structure at the distal end of the pre-embedded guidewire 710 and the branch guidewire 910 is released, such that the pre-embedded guidewire 710 is detached from the branch guidewire 910. Then the branch guidewire 910 is advanced from the other end 2002 of the arterial blood vessel 2000 to make the branch guidewire 910 move forwards and extend into the branch vessel 2100. Thereafter, as shown in FIG. 14, a branch stent 900 may be guided by the branch guidewire 910 to reach the branch vessel 2100 through the fenestration 811 of the main stent 800. The branch guidewire 910 communicates with the main stent 800.

In this embodiment, due to the guide of the pre-embedded guidewire 710, the branch guidewire 910 may easily pass through the fenestration 811 of the main stent 800. Thereafter, the branch guidewire 910 may be delivered further to easily enter into the branch vessel. Thereafter, the branch stent 900 may be easily pass through the fenestration 811 of the main stent 800 and then reach the branch vessel with the guide of the branch guidewire 910, thus, facilitating the delivery of the branch stent 900.

In this embodiment, as shown in FIG. 1, the proximal end of the delivery device 1000 is provided with a second locking assembly 720 which is configured to lock the movement of the pre-embedded guidewire 710. In this embodiment, the delivery device 1000 further includes a control handle 300, a rear fastener 640, a rear slideway 650. The control handle 300 is connected with a proximal end of the outer sheath 200. The rear fastener 640 is secured to a proximal end of the control handle 300. The rear slideway 650 is secured to a proximal end of the rear fastener 640. The second locking assembly 720 is provided on the rear slideway 650. The pre-embedded guidewire 710 passes through the rear slideway 650 and the rear fastener 640 and enters the delivering gap 210.

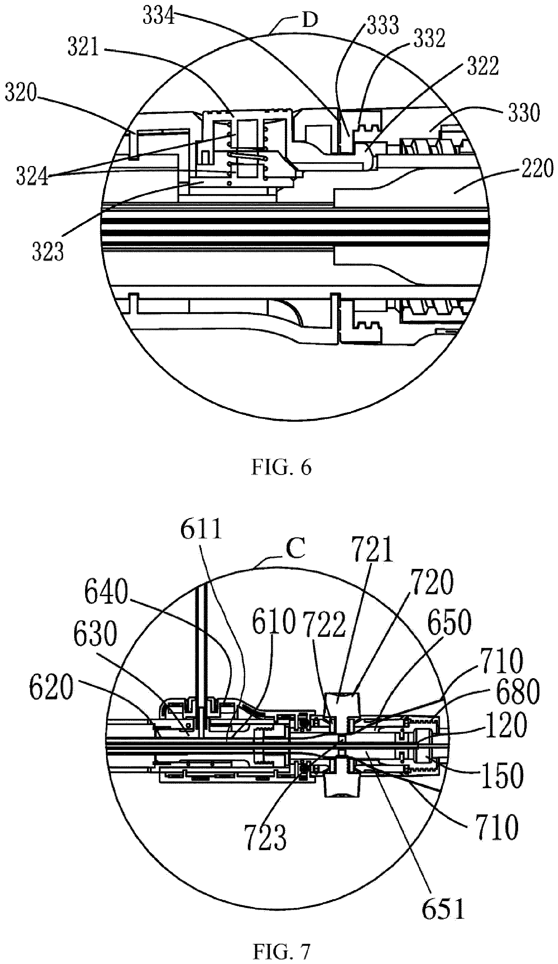

Specifically, as shown in FIG. 7, the rear slideway 650 defines therein a hollow rear sliding passage 651 extending in an axial direction of the rear slideway 650. The rear sliding passage 651 communicates with the delivering gap 210, and the pre-embedded guidewire 710 enters the rear sliding passage 651. The second locking assembly 720 includes a wire fastener 721, a fixed annular protrusion 722 secured on the rear slideway 650 and a blocking member 723 secured inside the rear sliding passage 651. The pre-embedded guidewire 710 enters the rear sliding passage 651 from a gap between the blocking member 723 and the wire fastener 721. Herein, the wire fastener 721 and the fixed annular protrusion 722 may be movably connected by a threaded connection. When the pre-embedded guidewire 710 is pressed tightly by the wire fastener 721 onto the blocking member 723, the movement of the pre-embedded guidewire 710 is locked, such that the pre-embedded guidewire 710 is prevented from being pulled to an improper position unintentionally.

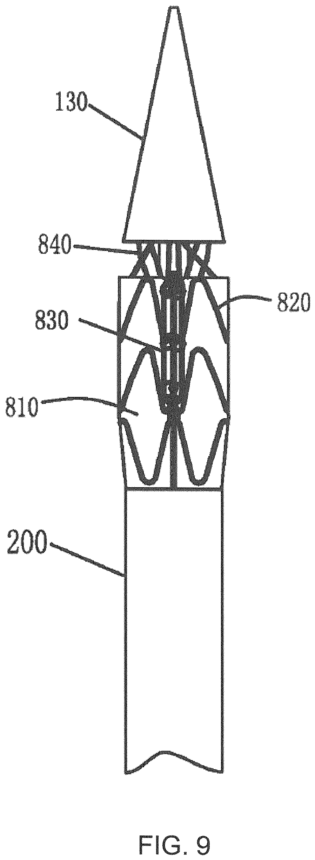

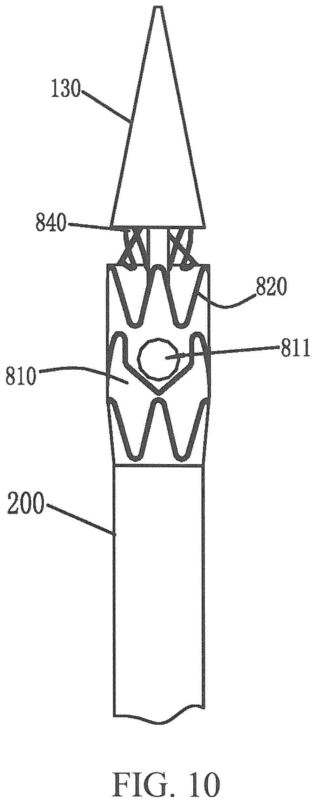

In this embodiment, as shown in FIGs. 1 and 2, the control handle 300 is connected with the outer sheath 200. The control handle 300 is configured to control the axial movement of the outer sheath 200 relative to the core assembly 100. Specifically, the control handle 300 is configured to control the outer sheath 200 to move axially relative to the outer tube 120. The axial direction refers to a direction extending between the left side and the right side in FIG. 1. With the control handle 300 controlling the outer sheath 200 to move axially, the folded main stent 800 in the delivering gap 210 is enabled to be in a partly released state or in a completely released state. Herein, referring to FIGs. 9 and 10, the partly released state refers to a state in which the proximal end of the main stent 800 is released and the released part of the main stent 800 is no more restrained by the outer sheath 200. As shown in FIG. 11, the completely released state refers to a state in which the whole main stent 800 is no more restrained by the outer sheath 200, that is the main stent 800 is not surrounded by the outer sheath 200. In this embodiment, the main stent 800 is released in a stepwise manner. Specifically, a part of the main stent 800 is released first, and in the meantime, the released part of the main stent 800 is exposed (as shown in FIGs. 9 and 10). Herein, the released part of the main stent 800 is located at a proximal end of the main stent 800, and the remaining part of the main stent 800 is remained within the delivery device 1000. At that time, the main stent 800 is in a partly released state. Then, a position of the main stent 800 is adjusted, and the remaining part of the main stent 800 may be released after the main stent 800 is adjusted in place. By that time, the main stent 800 is completely released (as shown in FIG. 11), i.e., in a completely released state. Alternatively, in other embodiment of the present disclosure, the main stent 800 may be released in more steps, not limited to two steps. In this embodiment, when the main stent 800 is in the partly released state, the fenestration 811 is located on the released part of the main stent 800.

In order to adjust an axial position of the outer sheath 200, in this embodiment, referring to FIGs. 1, 2 and 5, the outer sheath 200 is secured to a sheath joint 220. In this embodiment, an outer periphery of a proximal end of the outer sheath 200 is secured to the sheath joint 220. When the sheath joint 220 moves axially, the sheath joint 220 will drive the outer sheath 200 to move axially. The control handle 300 includes a support body 310, a fixed handle 320 and a sliding handle 330. The support body 310 is hollow and the sheath joint 220 is provided therein. When a force is applied to the sheath joint 220 in the axial direction, the sheath joint 220 moves axially within the support body 310 and drives the outer sheath 200 to move axially. The fixed handle 320 is mounted outside a distal end of the support body 310 and secured thereto. The sliding handle 330 is mounted outside the support body 310 adjacent to a proximal end of the fixed handle 320. The sliding handle 330 is rotatable relative to the support body 310 and drives the sheath joint 220 to move axially. That is, the rotation of the sliding handle 330 is transformed into the axial movement of the sheath joint 220.

Specifically, an elongated hole 311 is defined on the support body 310 and extends in the axial direction thereof. A tooth block 340 is provided outside the support body 310 at a position corresponding to the elongated hole 311. The sheath joint 220 includes a joint body 222, an abutment block 221 and a distal protrusion 223 that are provided on the joint body 222. The abutment block 221 and the distal protrusion 223 extend through the elongated hole 311 and abut against a proximal end and a distal end of the tooth block 340, respectively, so as to limit an axial movement of the tooth block 340 relative to the sheath joint 220. The sliding handle 330 is provided with an internal thread inside, and the tooth block 340 is provided with an external thread outside engaging with the internal thread. When the sliding handle 330 is rotated, the tooth block 340 is driven by the sliding handle 330 to move axially, and thus drives the abutment block 221 and the distal protrusion 223 to move in the axial direction within the elongated hole 311, and in turn the outer sheath 200 is driven to move axially, such that it is possible to enable the main stent 800 take the partly released state or take the completely released state.

In this embodiment, the sliding handle 330 is further axially slidable on the support body 310, that is, the sliding handle 330 is axially slidable relative to the fixed handle 320. In order to hold the sliding handle 330 axially in position to enhance the security of the operation, in this embodiment, as shown in FIG. 6, an unlocking button 321 is embedded in the fixed handle 320. A hook 322 extends from one side of the unlocking button 321. A button support 323 is provided below the unlocking button 321 and secured on the support body 310. Two positioning pillars 324 are provided between the unlocking button 321 and the button support 232 and arranged opposite to each other. A spring is mounted around the two positioning posts 324, disposed between the unlocking button 321 and the button support 232, and configured to urge the hook 322 in position. The hook 322 hooks the sliding handle 330, such that the sliding handle 330 is arranged next to the fixed handle 320 and prevented from axially sliding. Specifically, the sliding handle 330 includes a rotating knob 332 disposed at a distal end of the sliding handle 330. The rotating knob 332 has an inner flange 333, and the hook 322 may extend into the rotating knob 332 and engage with the inner flange 333. One side of the inner flange engaging with the hook 322 is defined as an annular positioning groove. That is, the hook 322 is positioned within the positioning groove, such that an axial position of the hook 322 is limited, while allowing the sliding handle 330 to rotate relative to the fixed handle 320. In this embodiment, two friction-reducing protruding rings 334 are coaxially provided on the rotating knob 332. The friction-reducing protruding rings 334 are disposed on a sidewall of a distal end of the rotating knob 322. Due to the friction-reducing protruding ring 334, contact between the fixed handle 320 and the sliding handle 330 is line contact instead of surface contact, which greatly reduces a contact area therebetween and thus reduces friction when they move relative to each other, resulting in a smoother and more accurate release of the main stent 800.

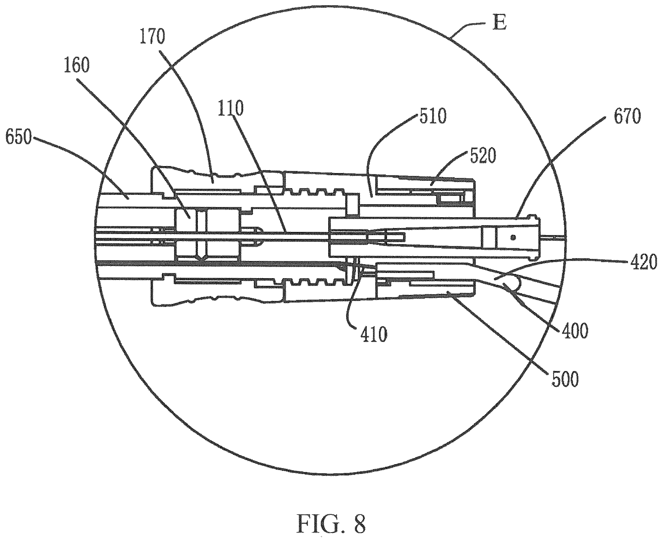

In order to prevent a problem in that a released part of the main stent 800 is fully unfolded when the main stent 800 is in the partly released state, which results in that the released part of the main stent 800 conform the arterial blood vessel 2000 tightly (the fully unfolded main stent 800 generally has a diameter generally about 10% larger than that of the blood vessel) and thus it is impossible to adjust the position of the main stent 800 when the main stent 800 has been released to an inaccurate position, in this embodiment, as shown in FIGs. 1 to 3 and 8, the delivery device 1000 includes a stent restraining assembly 400, which is configured to enable the released part of the main stent 800 to be partly unfolded when the main stent 800 is in the partly released state, so as to restrain the outer diameter of the released part of the main stent 800. Here, the released part of the main stent 800 refers to a part of the main stent 800 not restrained by the outer sheath 200, i.e., the part of the main stent 800 which is exposed from the outer sheath 200. Herein, a ratio of the outer diameter of the released part of the main stent 800 when folded to the outer diameter of the released part of the main stent 800 when unfolded is smaller than or equal to 90%. Since the released part of the main stent 800 is partly unfolded and the outer diameter thereof is relatively small, the released part of the main stent 800 would not tightly conform the blood vessel. In this way, when the main stent 800 is released to an inaccurate position, for example, the main stent 800 is inaccurately aligned in a circumferential direction, or the fenestration 811 (as shown in FIG. 10) on the main stent 800 is inaccurately positioned, without resistance between the released part of the main stent 800 and the blood vessel 2000, the delivery device 1000 can easily drive the main stent 800 to move, for example, rotate or move axially, so as to adjust the position of the main stent 800 conveniently, for example, to adjust the circumferential alignment of the main stent 800. After the main stent 800 is adjusted in place, the outer sheath 200 may be operated to move towards the proximal end of the delivery device 1000 in the axial direction to completely release the main stent 800, and the main stent 800 is fully unfolded and tightly conform the blood vessel 2000. The delivery device 1000 in this embodiment can be used to adjust the main stent 800 conveniently, which facilitates time saving and energy saving for the operator. In this embodiment, the stent restraining assembly 400 may make the released part of the main stent 800 be fully unfolded as soon as the main stent 800 is adjusted in place, or may make the main stent 800 be fully unfolded until the main stent 800 is in the completely released state. Additionally, in other embodiments of the present disclosure, the delivery device 1000 may be operated by the operator to adjust the position of the main stent 800 when the main stent 800 is inaccurately positioned in the axial direction.

In this embodiment, the stent restraining assembly 400 includes at least one control wire 410. Herein, one control wire, or two or more control wires 410 may be provided. The control wire 410 may be made of stainless steel. The control wire 410 enters the delivering gap 210 through the proximal end of the delivering gap 210 and extends to the distal end of the delivering gap 210.

As shown in FIG. 11, the main stent 800 includes a tubular membrane 810 and an annular support frame 820. A plurality of connecting members 830 are provided on the tubular membrane 810 and extend axially from a proximal end of the tubular membrane 810 towards a distal end of the tubular membrane 810. The connecting members 830 are arranged in at least two columns which are spaced apart in a circumferential direction of the tubular membrane 810. Preferably, the connecting member 830 has a proximal end located at the proximal end of the tubular membrane 810 and a distal end located at the middle of the tubular membrane 810. The tubular membrane 810 is provided with at least one fenestration 811 thereon, which is disposed between the proximal end and the middle of the tubular membrane 810. When the main stent 800 is in the partly released state, the tubular membrane 810 is released from its proximal end towards its distal end, but generally not beyond the middle of tubular membrane 810, and at that time the fenestration 811 is located on the released part of the main stent 800. The control wire 410 bounds at least two columns of the connecting members 830 together to restrain the main stent 800 to be partly unfolded, so as to restrain the outer diameter of the released part of the main stent 800. The outer diameter of the released part of the main stent 800 is smaller than the diameter of the blood vessel, which facilitates adjust the position of the main stent 800, for example, adjust the main stent 800 to make the fenestration 811 align with a branch vessel 2100. Preferably, a circumferential length between two columns of the connecting members 830 bounded by the control wire 410 may result in 10% or above reduction of the outer diameter of the released part of the main stent 800, that is, the ratio of the outer diameter of the released part of the main stent 800 when folded to the outer diameter of the released part of the main stent 800 when unfolded is smaller than or equal to 90%. The stent restraining assembly 400 releases the bound to the connecting members 830 to make the main stent 800 fully unfold. The specific structure of the main stent 800 in this embodiment has been discussed in detail in an early filed co-pending Chinese patent application No. 201711483955.7.

In this embodiment, as shown in FIGs. 1, 2 and 8, the stent restraining assembly 400 further includes a pull-tab 420 which is secured to a proximal end of the control wire 410, to facilitate the operation of the control wire 410. Specifically, a force applied to the proximal end of the delivery device 1000 by the operator through the pull-tab 420 may release the bound of the control wire 410 to the main stent 800, so as to achieve unfolding of the restrained part of the main stent 800.

In order to improve the security for use of the device, in this embodiment, the delivery device 1000 further includes a first locking assembly 500 configured to lock the movement of the stent restraining assembly 400, so as to avoid releasing the restraining of the control wire 410 to the main stent 800 unintentionally. In this embodiment, the first locking assembly 500 is configured to lock the movement of the pull-tab 420, so as to avoid releasing the restraining of the control wire 410 to the main stent 800 unintentionally. In addition, in other embodiments of the present disclosure, the first locking assembly 500 may be configured to lock the movement of the control wire 410, so as to avoid releasing the restraining of the control wire 410 to the main stent 800. In this embodiment, the first locking assembly 500 includes a pull-tab fastener 510 and a proximal releasing knob 520. The pull-tab fastener 510 is connected to a proximal end of a rear slideway 650 (described hereafter) by a threaded connection. The pull-tab fastener 510 defines a through hole configured for positioning the pull-tab 420, and a first groove for engaging with the proximal releasing knob 520. The proximal releasing knob 520 is rotatably mounted at a proximal end of the pull-tab fastener 510. An L-shaped protrusion is provided inside a distal end of the proximal releasing knob 520, which has a relatively long arm extending in a circumferential direction of the proximal releasing knob 520 and a relatively short arm extending from one end of the relative long arm towards the distal end in an axial direction of the proximal releasing knob 520, so as to define a second groove. A second protrusion is provided at a distal end of the pull-tab 420 and extends outwards. After the pull-tab 420 is inserted in the through hole and secured therein, the proximal releasing knob 520 is rotated to make the second protrusion enter into the second groove, such that the pull-tab 420 is locked there. In this way, the pull-tab 420 is unable to drive the control wire 410 to move towards the proximal end, so as to avoid releasing the restraining to the main stent 800 unintentionally. When the proximal releasing knob 520 is rotated such that the second protrusion is disengaged from the second groove, the pull-tab 420 is able to be operated to drive the control wire 410 to move towards the proximal end, so as to release the restraining to the main stent 800.

In this embodiment, referring to FIGs 1 to 3, and 7, the delivery device 1000 further includes a push-rod 610 and a support tube 620. The support tube 620 is disposed between the outer sheath 200 and the outer tube 120. That is, the support tube 620 is at least partly disposed in the delivering gap 210. The support tube 620 serves to enhance the support. The push-rod 610 is disposed between the support tube 620 and the outer tube 120, with a distal end thereof disposed in the delivering gap 210. When the control handle 300 controls the outer sheath 200 to axially move relative to the core assembly 100 towards the proximal end, the distal end of the push-rod 610 is configured to abut against the main stent 800 so as to prevent the main stent 800 from moving towards the proximal end of the delivering gap 210. In the meanwhile, the push-rod 610 serves to support the main stent 800. In addition, in this embodiment, the push-rod 610 defines a through passage 611 inside in an axial direction. The through passage 611 is configured to allow the control wire 410 and the pre-embedded guidewire 710 to pass therethrough.

In this embodiment, the delivery device 1000 further includes a push-rod fastener 630 and a rear fastener 640. The push-rod fastener 630 is fixedly connected to the proximal end of the push-rod 610 and the support tube 620. The push-rod fastener 630 has a distal end disposed at an inner side of a proximal end of the support body 310 and secured to the proximal end of the support body 310. The rear fastener 640 is mounted around an outer periphery of the proximal end of the support body 310, and has a distal end secured to the proximal end of the support body 310. In addition, a connecting port extends out from the push-rod fastener 630, which is configured to be connected to an end of a TPU flexible tube 3001. Another end of the TPU flexible tube 3001 is connected with a three-way valve 3002.

In this embodiment, the rear fastener 640 has a proximal end secured to the rear slideway 650 which has a distal end disposed at an inner side of the rear fastener 640. The rear slideway 640 is provided therein with an outer tube fastener 150, which is disposed at a position in proximity to the middle of the rear slideway 650 and secured to an outer periphery of the proximal end of the outer tube 120. An outer cover 680 is provided adjacent to the proximal end of the rear slideway 650 and mounted around the rear slideway 650.

In this embodiment, as shown in FIG. 1 to 3, 7 and 8, an inner core securing steel sleeve 160 and a rear releasing knob 170 are provided at the proximal end of the rear slideway 650. The inner core securing steel sleeve 160 is disposed inside the rear slideway 650. The inner core securing steel sleeve 160 is secured to the inner core 110 and disposed outside the inner core 110. The rear releasing knob 170 is disposed outside the proximal end of the rear slideway 650. The rear releasing knob 170 is secured to the inner core securing steel sleeve 160. An elongated hole is defined at the proximal end of the rear slideway 650 which extends in an axial direction. The rear releasing knob 170 can drive the inner core securing steel sleeve 160 to move in the axial direction within the elongated hole, so as to control a rear release of the main stent 800. In this embodiment, the proximal end of the rear slideway 650 is secured to the distal end of the pull-tab fastener 510. In this embodiment, the inner core 110 has a proximal end secured to a luer joint 670.

It should be noted that each embodiment in this specification is described in a progressive manner and focuses on the differences from the other embodiments, and the same parts between those embodiments may be referred to each other.

## Claims

1. A delivery device (1000) configured to deliver a main stent (800) having a fenestration (811), **characterized in that**, the delivery device (1000) comprises:
a core assembly (100);
an outer sheath (200), which is hollow and mounted around an outer periphery of the core assembly (100), with a delivering gap (210) defined between the outer sheath (200) and the core assembly (100), wherein the delivering gap (210) has a distal end for receiving a folded main stent (800); and
a pre-embedded guidewire (710), entering the delivering gap (210) through the proximal end of the delivering gap (210) and extending to the distal end of delivering gap passage, the pre-embedded guidewire (710) having a distal end configured to enter into the main stent (800) from an outside of the main stent (800) via the fenestration (811), and the pre-embedded guidewire (710) being configured to guide a branch guidewire (910) to pass through the main stent (800) from an inside to the outside of the main stent (800) via the fenestration (811) of the main stent (800), wherein
the delivery device (1000) further comprises a control handle (300) connected with the outer sheath (200), and wherein the control handle (300) controls the outer sheath (200) to move axially relative to the core assembly (100) so as to enable the folded main stent (800) to be in a partly released state or in a completely released state, and
a sheath joint (220) is secured to an outer periphery of the outer sheath (200), and the control handle (300) comprises:
a support body (310), with the sheath joint (220) provided therein, wherein when being subjected to an axial force, the sheath joint (220) moves axially within the support body (310) to drive the outer sheath (200) to move axially;
a fixed handle (320), mounted outside a distal end of the support body (310) and secured thereto; and
a sliding handle (330), mounted outside a distal end of the support body (310) adjacent to a proximal end of the fixed handle (320), the sliding handle (330) being rotatable at the outside of the support body (310) so as to drive the sheath joint (220) to move axially, and
the sliding handle (330) is axially slidable on the support body (310), an unlocking button (321) is embedded in the fixed handle (320), the unlocking button (321) is provided with a hook (322) extending from one side of the unlocking button (321), and the hook (322) hooks the sliding handle (330) in such a way that the sliding handle (330) is arranged next to the fixed handle (320) and prevented from axially sliding.

2. The delivery device (1000) according to claim 1, wherein a locking assembly (720) is provided at a proximal end of the delivery device (1000) and configured to lock a movement of the pre-embedded guidewire (710).

3. The delivery device (1000) according to claim 2, wherein the delivery device (1000) further comprises:
the control handle (300), connected with a proximal end of the outer sheath (200);
a rear fastener (640), secured to a proximal end of the control handle (300); and
a rear slideway (650), secured to a proximal end of the rear fastener (640), wherein the locking assembly (720) is provided on the rear slideway (650), and the pre-embedded guidewire (710) passes through the rear slideway (650) and the rear fastener (640) and enters the delivering gap (210).

4. The delivery device (1000) according to claim 3, wherein the rear slideway (650) defines therein a hollow rear sliding passage (651) extending in an axial direction of the rear slideway (650), the rear sliding passage (651) communicates with the delivering gap (210); the locking assembly (720) comprises a wire fastener (721), a fixed annular protrusion (722) secured on the rear slideway (650) and a blocking member (723) secured inside the rear sliding passage (651), the pre-embedded guidewire (710) enters the rear sliding passage (651) from a gap between the wire fastener (721) and the blocking member (723), the wire fastener (721) and the fixed annular protrusion (722) are connected by a threaded connection, and wherein when the wire fastener (721) presses tightly the pre-embedded guidewire (710) onto the blocking member (723), the movement of the pre-embedded guidewire (710) is locked.

5. The delivery device (1000) according to claim 1, wherein the delivery device (1000) further comprises a stent restraining assembly (400) configured to restrain a released part of the main stent (800) to be partly unfolded when the main stent (800) is in the partly released state in such a way to restrain an outer diameter of the released part of the main stent (800), and to make the main stent (800) be fully unfolded when the main stent (800) is in the completely released state.

6. The delivery device (1000) according to claim 5, wherein the stent restraining assembly (400) comprises at least one control wire (410), and the control wire (410) enters the delivering gap (210) through a proximal end of the delivering gap (210) and extending to the distal end of the delivering gap (210), and wherein the control wire (410) has a distal end configured to circumferentially restrain the released part of the main stent (800).

7. The delivery device (1000) according to claim 6, wherein the stent restraining assembly (400) further comprises a pull-tab (420) secured to a proximal end of the control wire (410), the pull-tab (420) is configured to apply a force to a proximal end of the delivery device (1000) so as to release the restraining of the control wire (410) to the main stent (800).

8. The delivery device (1000) according to claim 5, wherein the delivery device (1000) further comprises another locking assembly (500) configured to lock the movement of the stent restraining assembly (400) to avoid releasing the restraining of the control wire (410) to the main stent (800) unintentionally.

9. The delivery device (1000) according to claim 1, wherein an elongated hole (311) is defined on the support body (310) and extends in an axial direction, a tooth block (340) is mounted outside the support body (310) at a position corresponding to the elongated hole (311), the sheath joint (220) comprises a joint body (222), and an abutment block (221) and a distal protrusion (223) provided on the joint body (222), the abutment block (221) and the distal protrusion (223) extend through the elongated hole (311) and abut against a proximal end and a distal end of the tooth block (340), respectively, in such a way to limit an axial movement of the tooth block (340) relative to the sheath joint (220), the sliding handle (330) is provided with an internal thread, the tooth block (340) is provided with an external thread engaging with the internal thread, and wherein when the sliding handle (330) rotates, the sliding handle (330) drives the sheath joint (220) to move axially through the tooth block (340), the abutment block (221) and the distal protrusion (223), and in turn drives the outer sheath (200) to move axially.

10. The delivery device (1000) according to claim 1, wherein the delivery device (1000) further comprises a push-rod (610), wherein the push-rod (610) has a distal end disposed in the delivering gap (210), and the push-rod (610) is configured to abut against the main stent (800) when the control handle (300) controls the outer sheath (200) to axially move relative to the core assembly (100) towards the proximal end, so as to prevent the main stent (800) from moving towards the proximal end of the delivering gap (210).

11. The delivery device (1000) according to claim 10, wherein the delivery device (1000) further comprises a support tube (620), wherein the support tube (620) has a distal end disposed in the delivering gap (210), and the push-rod (610) is disposed in the support tube (620).

12. The delivery device (1000) according to claim 10, wherein the push-rod (610) defines therein a through passage (611) extending in an axial direction thereof, and the pre-embedded guidewire (710) enters the through passage (611) from a proximal end thereof and extends out of the through passage (611) from a distal end thereof.

13. The delivery device (1000) according to claim 1, wherein the sliding handle (330) includes a rotating knob (332) disposed at a distal end of the sliding handle (330), wherein the rotating knob (332) has an inner flange (333) and the hook (322) extends into the rotating knob (332) and engages with the inner flange (333), wherein one side of the inner flange (333) engaging with the hook (322) is defined as an annular positioning groove.

14. The delivery device (1000) according to claim 13, wherein a button support (323) is provided below the unlocking button (321) and secured on the support body (310), and a spring is disposed between the unlocking button (321) and the button support (232) and is configured to urge the hook (322) in position.

15. The delivery device (1000) according to claim 14, wherein two positioning posts (324) are provided between the unlocking button (321) and the button support (232) and are arranged opposite to each other, and the spring is mounted around the two positioning posts (324).

## Patentansprüche

1. Eine Zuführvorrichtung (1000) die konfiguriert ist, um einen Hauptstent (800) zuzuführen, der eine Fensterung (811) aufweist, **dadurch gekennzeichnet, dass** die Zuführvorrichtung (1000) umfasst:
eine Kernanordnung (100);
eine äußere Hülse (200), die hohl ist und um einen äußeren Umfang der Kernanordnung (100) montiert ist, wobei ein Zuführspalt (210) zwischen der äußeren Hülse (200) und der Kernanordnung (100) definiert ist, wobei der Zuführspalt (210) ein distales Ende zum Aufnehmen eines zusammengefalteten Hauptstents (800) aufweist; und
einen vorab eingebetteten Führungsdraht (710), der in den Zuführspalt (210) durch das proximale Ende des Zuführspalts (210) tritt und sich zu dem distalen Ende des Zuführspaltabschnitts erstreckt, wobei der vorab eingebettete Führungsdraht (710) ein distales Ende aufweist, das konfiguriert ist, um in den Hauptstent (800) von einer Außenseite des Hauptstents (800) über die Fensterung (811) einzutreten, und wobei der vorab eingebettete Führungsdraht (710) konfiguriert ist, um einen abgezweigten Führungsdraht (910) zu leiten, sodass dieser durch den Hauptstent (800) von einer Innenseite zu der Außenseite des Hauptstents (800) über die Fensterung (811) des Hauptstents (800) tritt, wobei die Zuführvorrichtung (1000) des Weiteren einen Steuergriff (300) aufweist, der mit der äußeren Hülse (200) verbunden ist, und wobei der Steuergriff (300) die äußere Hülse (200) so steuert, dass sie sich axial bezogen auf die Kernanordnung (100) bewegt, um es so dem zusammengefalteten Hauptstent (800) zu ermöglichen, sich in einem teilweise freigegebenen Zustand oder in einem vollständig freigegebenen Zustand zu befinden, und
ein Hülsengelenk (220) ist an einem äußeren Umfang der äußeren Hülse (200) befestigt, und der Steuergriff (300) umfasst:
einen Stützkörper (310), in dem das Hülsengelenk (220) vorgesehen ist, wobei dann, wenn das Hülsengelenk (220) einer axialen Kraft ausgesetzt ist, es sich axial innerhalb des Stützkörpers (310) bewegt, um die äußere Hülse (200) zu veranlassen, sich axial zu bewegen;
einen festen Griff (320), der außerhalb eines distalen Endes des Stützkörpers (310) angeordnet ist und daran befestigt ist; und
einen Schiebegriff (330), der außerhalb eines distalen Endes des Stützkörpers (310) benachbart zu einem proximalen Ende des festen Griffs (320) angeordnet ist, wobei der Schiebegriff (330) an der Außenseite des Stützkörpers (310) drehbar ist, um das Hülsengelenk (220) so anzutreiben, dass es sich axial bewegt, und der Schiebegriff (330) ist axial gleitbar auf dem Stützkörper (310), ein Entriegelungsknopf (321) ist in dem festen Griff eingebettet, der Entriegelungsknopf (321) ist mit einem Haken (322) versehen, der sich von einer Seite des Entriegelungsknopfs (321) erstreckt, und der Haken (322) hakt so in den Schiebegriff (330) ein, dass der Schiebegriff (330) neben dem festen Griff (320) angeordnet ist und an einem axialen Verschieben gehindert wird.

2. Die Zuführvorrichtung (1000) gemäß Anspruch 1, wobei eine Verriegelungsanordnung (720) an einem proximalen Ende der Zufuhrvorrichtung (1000) vorgesehen und so konfiguriert ist, dass sie eine Bewegung des vorab eingebetteten Führungsdrahtes (710) sperrt.

3. Die Zufuhrvorrichtung (1000) gemäß Anspruch 2, wobei die Zuführvorrichtung (1000) weiterhin umfasst:
den Steuergriff (300), der mit einem proximalen Ende der äußeren Hülse (200) verbunden ist;
eine hintere Befestigung (640), die an einem proximalen Ende des Steuergriffs (300) befestigt ist; und
eine hintere Gleitbahn (650), die an einem proximalen Ende der hinteren Befestigung (640) befestigt ist, wobei die Verriegelungsanordnung (720) an der hinteren Gleitbahn (650) vorgesehen ist, und der vorab eingebettete Führungsdraht (710) die hintere Gleitbahn (650) und die hintere Befestigung (640) passiert und in den Zuführspalt (210) tritt.

4. Die Zuführvorrichtung (1000) gemäß Anspruch 3, wobei die hintere Gleitbahn (650) darin einen hohlen hinteren Gleitdurchgang (651) definiert, der sich in einer axialen Richtung der hinteren Gleitbahn (650) erstreckt, der hintere Gleitdurchgang (651) steht mit dem Zuführspalt (210) in Verbindung; die Verriegelungsanordnung (720) umfasst eine Drahtbefestigung (721), einen festen ringförmigen Vorsprung (722), der an der hinteren Gleitbahn (650) befestigt ist und ein Blockierelement (723), das im Inneren des hinteren Gleitdurchgangs (651) befestigt ist, der vorab eingebettete Führungsdraht (710) tritt über einen Spalt zwischen der Drahtbefestigung (721) und dem Blockierelement (723) in den hinteren gleitenden Durchgang ein, die Drahtbefestigung (721) und der feste ringförmige Vorsprung (722) sind über eine Gewindeverbindung verbunden, und wobei dann, wenn die Drahtbefestigung (721) den vorab eingebetteten Führungsdraht (710) fest auf das Blockierelement (723) presst, die Bewegung auf dem vorab eingebetteten Führungsdraht (710) gesperrt ist.

5. Die Zuführvorrichtung (1000) gemäß Anspruch 1, wobei die Zuführvorrichtung (1000) des Weiteren eine Stentrückhalteanordnung (400) umfasst, die so konfiguriert ist, dass sie einen freigegebenen Teil des Hauptstents (800) davor zurückhält, zum Teil aufgefaltet zu werden, wenn sich der Hauptstent (800) in dem teilweise freigegebenen Zustand befindet, derart, dass ein äußerer Umfang des gelösten Teils des Hauptstents (800) zurückgehalten wird, und den Hauptstent (800) dazu zu bringen, vollständig auseinander gefaltet zu sein, wenn sich der Hauptstent in dem vollständig freigegebenen Zustand befindet.

6. Die Zuführvorrichtung (1000) gemäß Anspruch 5, wobei die Stentrückhalteanordnung (400) mindestens einen Steuerdraht (410) umfasst, und der Steuerdraht (410) tritt durch ein proximales Ende des Zuführspalts (210) in den Zufuhrspalt (210) und erstreckt sich zu dem distalen Ende des Zufuhrspalts (210), und wobei der Steuerdraht (410) ein distales Ende aufweist, das konfiguriert ist, um den freigegebenen Teil des Hauptstents (800) umfänglich zurückzuhalten.

7. Die Zuführvorrichtung (1000) gemäß Anspruch 6, wobei die Stentrückhalteanordnung (400) des Weiteren eine Zuglasche (420) umfasst, die an einem proximalen Ende des Steuerdrahts (410) befestigt ist, die Zuglasche (420) ist so konfiguriert, dass sie eine Kraft auf ein proximales Ende der Zuführvorrichtung (1000) ausübt, um die Rückhaltung des Steuerdrahts (410) auf den Hauptstent (800) freizugeben.

8. Die Zuführvorrichtung (1000) gemäß Anspruch 5, wobei die Zuführvorrichtung (1000) des Weiteren eine andere Verriegelungsanordnung (500) aufweist, die so konfiguriert ist, dass sie die Bewegung der Stentrückhalteanordnung (400) sperrt, um ein unbeabsichtigtes Freigeben der Rückhaltung des Steuerdrahts (410) auf den Hauptstent (800) zu verhindern.

9. Die Zuführvorrichtung (1000) gemäß Anspruch 1, wobei ein Langloch (311) auf dem Stützkörper (310) definiert ist und sich in einer axialen Richtung erstreckt, ein Zahnblock (340) ist außerhalb des Stützkörpers (310) an einer Position passend zu dem Langloch (311) montiert, das Hülsengelenk (220) umfasst einen Gelenkkörper (222) und einen Anschlagblock (221) und einen distalen Vorsprung (223) die an dem Gelenkkörper (222) vorgesehen sind, der Anschlagblock (221) und der distale Vorsprung (223) erstrecken sich durch das Langloch (311) und stoßen an ein proximales Ende bzw. ein distales Ende des Zahnblocks (340) derart an, dass sie eine axiale Bewegung des Zahnblocks (340) bezogen auf das Hülsengelenk (220) einschränken, der Schiebegriff (330) ist mit einem Innengewinde versehen, der Zahnblock (340) ist mit einem Außengewinde versehen, das in das Innengewinde eingreift, und wobei dann, wenn sich der Schiebegriff (330) dreht, der Schiebegriff (330) das Hülsengelenk (220) veranlasst, sich axial durch den Zahnblock (340), den Anschlagblock (221) und den distalen Vorsprung (223) zu bewegen, und wiederum die Außenhülse (200) veranlasst, sich axial zu bewegen.

10. Die Zuführvorrichtung (1000) gemäß Anspruch 1, wobei die Zuführvorrichtung (1000) des Weiteren eine Schubstange (610) umfasst, wobei die Schubstange (610) ein distales Ende aufweist, das in dem Zuführspalt (210) aufgenommen ist, und die Schubstange (610) ist so konfiguriert, dass sie an den Hauptstent (800) anstößt, wenn der Steuergriff (300) die Außenhülse (200) so steuert, dass sie sich axial bezogen auf die Kernanordnung (100) in Richtung des proximalen Endes bewegt, um den Hauptstent (800) daran zu hindern, sich in Richtung des proximalen Endes des Zuführspalts (210) zu bewegen.

11. Die Zuführvorrichtung (1000) gemäß Anspruch 10, wobei die Zuführvorrichtung (1000) des Weiteren ein Stützrohr (620) umfasst, wobei das Stützrohr (620) ein distales Ende aufweist, das in dem Zuführspalt (210) aufgenommen ist, und die Schubstange (610) ist in dem Stützrohr (620) aufgenommen.

12. Die Zuführvorrichtung (1000) gemäß Anspruch 10, wobei die Schubstange (610) darin eine Durchgangspassage (611) definiert, die sich in einer axialen Richtung davon erstreckt, und der vorab eingebettete Führungsdraht (710) tritt von einem proximalen Ende davon in die Durchgangspassage (611) ein und erstreckt sich von einem distalen Ende davon aus der Durchgangspassage (611) heraus.

13. Die Zuführvorrichtung (1000) gemäß Anspruch 1, wobei der Schiebegriff (330) einen Drehgriff (332) umfasst, der an einem distalen Ende des Schiebegriffs (330) angebracht ist, wobei der Drehgriff (332) einen inneren Flansch (333) aufweist und der Haken (322) sich in den Drehgriff (332) erstreckt und mit dem inneren Flansch (333) in Eingriff gelangt, wobei eine Seite des inneren Flansches (333), die mit dem Haken (322) in Eingriff gelangt, als eine ringförmige Positionsnut definiert ist.

14. Die Zuführvorrichtung (1000) gemäß Anspruch 13, wobei eine Knopfhalterung (323) unterhalb des Entriegelungsknopfes (321) vorgesehen ist und an dem Stützkörper (310) befestigt ist, und eine Feder ist zwischen dem Entriegelungsknopf (321) und der Knopfhalterung (232) angeordnet und ist konfiguriert, den Haken (322) in Position zu drängen.

15. Die Zuführvorrichtung (1000) gemäß Anspruch 14, wobei zwei Positionierungssäulen (324) zwischen dem Entriegelungsknopf (321) und der Knopfstütze (232) vorgesehen sind und einander gegenüberliegend angeordnet sind, und die Feder ist um die zwei Positionierungssäulen (324) herum montiert.

## Revendications

1. Dispositif de pose (1000) configuré pour poser une endoprothèse principale (800) comportant une fenestration (811), **caractérisé en ce que** le dispositif de pose (1000) comprend :
un ensemble de noyau (100) ;
une enveloppe extérieure (200), laquelle est creuse et montée autour d'une périphérie extérieure de l'ensemble de noyau (100), avec un espace de pose (210) défini entre l'enveloppe extérieure (200) et l'ensemble de noyau (100), dans lequel l'espace de pose (210) présente une extrémité distale destinée à recevoir une endoprothèse principale (800) pliée ; et
un fil de guidage pré-incorporé (710), entrant dans l'espace de pose (210) à travers l'extrémité proximale de l'espace de pose (210) et s'étendant vers l'extrémité distale du passage d'espace de pose, le fil de guidage pré-incorporé (710) présentant une extrémité distale configurée pour entrer dans l'endoprothèse principale (800) depuis un extérieur de l'endoprothèse principale (800) via la fenestration (811), et le fil de guidage pré-incorporé (710) étant configuré pour guider un fil de guidage de dérivation (910) destiné à passer à travers l'endoprothèse principale (800) depuis un intérieur vers l'extérieur de l'endoprothèse principale (800) via la fenestration (811) de l'endoprothèse principale (800), dans lequel
le dispositif de pose (1000) comprend en outre un manche de commande (300) relié à l'enveloppe extérieure (200), et dans lequel le manche de commande (300) commande l'enveloppe extérieure (200) pour la déplacer axialement par rapport à l'ensemble de noyau (100) de manière à permettre à l'endoprothèse principale (800) pliée d'être dans un état partiellement libéré ou dans un état complètement libéré, et
un joint d'enveloppe (220) est fixé à une périphérie extérieure de l'enveloppe extérieure (200), et le manche de commande (300) comprend :
un corps de support (310), avec le joint d'enveloppe (220) disposé dans celui-ci, dans lequel, lorsqu'il est soumis à une force axiale, le joint d'enveloppe (220) se déplace axialement à l'intérieur du corps de support (310) pour amener l'enveloppe extérieure (200) à se déplacer axialement ;
un manche fixe (320), monté à l'extérieur d'une extrémité distale du corps de support (310) et fixé à celle-ci ; et
un manche coulissant (330), monté à l'extérieur d'une extrémité distale du corps de support (310) adjacente à une extrémité proximale du manche fixe (320), le manche coulissant (330) étant rotatif à l'extérieur du corps de support (310) de manière à amener le joint d'enveloppe (220) à se déplacer axialement, et
le manche coulissant (330) peut coulisser axialement sur le corps de support (310), un bouton de déverrouillage (321) est incorporé dans le manche fixe (320), le bouton de déverrouillage (321) est doté d'un crochet (322) s'étendant à partir d'un côté du bouton de déverrouillage (321), et le crochet (322) accroche le manche coulissant (330) de telle façon que le manche coulissant (330) est agencé à côté du manche fixe (320) et empêché de coulisser axialement.

2. Dispositif de pose (1000) selon la revendication 1, dans lequel un ensemble de verrouillage (720) est disposé à une extrémité proximale du dispositif de pose (1000) et configuré pour verrouiller un déplacement du fil de guidage pré-incorporé (710).

3. Dispositif de pose (1000) selon la revendication 2, dans lequel le dispositif de pose (1000) comprend en outre :
la manche de commande (300), relié à une extrémité proximale de l'enveloppe extérieure (200) ;
un moyen de fixation arrière (640), fixé à une extrémité proximale du manche de commande (300) ; et
une voie de coulissement arrière (650), fixée à une extrémité proximale du moyen de fixation arrière (640), dans lequel l'ensemble de verrouillage (720) est disposé sur la voie de coulissement arrière (650), et le fil de guidage pré-incorporé (710) passe à travers la voie de coulissement arrière (650) et le moyen de fixation arrière (640) et entre dans l'espace de pose (210).

4. Dispositif de pose (1000) selon la revendication 3, dans lequel la voie de coulissement arrière (650) définit un passage de coulissement arrière creux (651) dans celle-ci, lequel s'étend dans une direction axiale de la voie de coulissement arrière (650), le passage de coulissement arrière (651) communique avec l'espace de pose (210) ; l'ensemble de verrouillage (720) comprend un moyen de fixation de fil (721), une saillie annulaire fixe (722) fixée à la voie de coulissement arrière (650) et un élément de blocage (723) fixé à l'intérieur du passage de coulissement arrière (651), le fil de guidage pré-incorporé (710) entre dans le passage de coulissement arrière (651) depuis un espace entre le moyen de fixation de fil (721) et l'élément de blocage (723), le moyen de fixation de fil (721) et la saillie annulaire fixe (722) sont reliés par un raccord fileté, et dans lequel, lorsque le moyen de fixation de fil (721) presse fermement le fil de guidage pré-incorporé (710) sur l'élément de blocage (723), le déplacement du fil de guidage pré-incorporé (710) est verrouillé.

5. Dispositif de pose (1000) selon la revendication 1, dans lequel le dispositif de pose (1000) comprend en outre un ensemble de retenue d'endoprothèse (400) configuré pour empêcher une partie libérée de l'endoprothèse principale (800) d'être partiellement dépliée lorsque l'endoprothèse principale (800) est dans l'état partiellement libéré, de manière à limiter un diamètre extérieur de la partie libérée de l'endoprothèse principale (800), et à amener l'endoprothèse principale (800) à être entièrement dépliée lorsque l'endoprothèse principale (800) est dans l'état complètement libéré.

6. Dispositif de pose (1000) selon la revendication 5, dans lequel l'ensemble de limitation d'endoprothèse (400) comprend au moins un fil de commande (410), et le fil de commande (410) entre dans l'espace de pose (210) à travers une extrémité proximale de l'espace de pose (210) et s'étend vers l'extrémité distale de l'espace de pose (210), et dans lequel le fil de commande (410) présente une extrémité distale configurée pour limiter circonférentiellement la partie libérée de l'endoprothèse principale (800).

7. Dispositif de pose (1000) selon la revendication 6, dans lequel l'ensemble de limitation d'endoprothèse (400) comprend en outre une languette (420) fixée à une extrémité proximale du fil de commande (410), la languette (420) est configurée pour appliquer une force à une extrémité proximale du dispositif de pose (1000) de manière à libérer l'action de limitation du fil de commande (410) sur l'endoprothèse principale (800).

8. Dispositif de pose (1000) selon la revendication 5, dans lequel le dispositif de pose (1000) comprend en outre un autre ensemble de verrouillage (500) configuré pour verrouiller le déplacement de l'ensemble de limitation d'endoprothèse (400) afin d'éviter la libération accidentelle de l'action de limitation du fil de commande (410) sur l'endoprothèse principale (800).

9. Dispositif de pose (1000) selon la revendication 1, dans lequel un trou oblong (311) est défini sur le corps de support (310) et s'étend dans une direction axiale, un bloc denté (340) est monté à l'extérieur du corps de support (310) à une position correspondant au trou oblong (311), le joint d'enveloppe (220) comprend un corps de joint (222), ainsi qu'un bloc de butée (221) et une saillie distale (223) disposés sur le corps de joint (222), le bloc de butée (221) et la saillie distale (223) s'étendent à travers le trou oblong (311) et butent contre une extrémité proximale et une extrémité distale du bloc denté (340), respectivement, de manière à restreindre un déplacement axial du bloc denté (340) par rapport au joint d'enveloppe (220), le manche coulissant (330) est doté d'un filetage interne, le bloc denté (340) est doté d'un filetage externe s'engageant avec le filetage interne, et dans lequel, lors de la rotation du manche coulissant (330), le manche coulissant (330) amène le joint d'enveloppe (220) à se déplacer axialement à travers le bloc denté (340), le bloc de butée (221) et la saillie distale (223), et amène à son tour l'enveloppe extérieure (200) à se déplacer axialement.

10. Dispositif de pose (1000) selon la revendication 1, dans lequel le dispositif de pose (1000) comprend en outre une tige de poussée (610), dans lequel la tige de poussée (610) présente une extrémité distale disposée dans l'espace de pose (210), et la tige de poussée (610) est configurée pour buter contre l'endoprothèse principale (800) lorsque le manche de commande (300) commande l'enveloppe extérieure (200) de manière à ce que celle-ci se déplace axialement par rapport à l'ensemble de noyau (100) vers l'extrémité proximale, de façon à empêcher l'endoprothèse principale (800) de se déplacer vers l'extrémité proximale de l'espace de pose (210).

11. Dispositif de pose (1000) selon la revendication 10, dans lequel le dispositif de pose (1000) comprend en outre un tube de support (620), dans lequel le tube de support (620) présente une extrémité distale disposée dans l'espace de pose (210), et la tige de poussée (610) est disposée dans le tube de support (620).

12. Dispositif de pose (1000) selon la revendication 10, dans lequel la tige de poussée (610) définit un passage traversant (611) en son intérieur, lequel s'étend dans une direction axiale de celle-ci, et le fil de guidage pré-incorporé (710) entre dans le passage traversant (611) depuis une extrémité proximale de celui-ci et s'étend hors du passage traversant (611) depuis une extrémité distale de celui-ci.

13. Dispositif de pose (1000) selon la revendication 1, dans lequel le manche coulissant (330) inclut un bouton rotatif (332) disposé à une extrémité distale du manche coulissant (330), dans lequel le bouton rotatif (332) comporte un rebord intérieur (333) et le crochet (322) s'étend dans le bouton rotatif (332) et s'engage avec le rebord intérieur (333), dans lequel un côté du rebord intérieur (333) s'engageant avec le crochet (322) est défini comme une rainure de positionnement annulaire.

14. Dispositif de pose (1000) selon la revendication 13, dans lequel un support de bouton (323) est prévu sous le bouton de déverrouillage (321) et fixé sur le corps de support (310), et un ressort est disposé entre le bouton de déverrouillage (321) et le support de bouton (232) et configuré pour pousser le crochet (322) en position.

15. Dispositif de pose (1000) selon la revendication 14, dans lequel il est prévu deux montants de positionnement (324) entre le bouton de déverrouillage (321) et le support de bouton (232), lesquels sont agencés de façon opposée l'un par rapport à l'autre, et le ressort est monté autour des deux montants de positionnement (324).
